# EUROPÄISCHE PATENTSCHRIFT

(11) **EP 0 547 414 B1**
(45) Veröffentlichungstag und Bekanntmachung des Hinweises auf die Patenterteilung: **20.03.1996**
(21) Anmeldenummer: 92120372.5
(22) Anmeldetag: 28.11.1992
(51) Int. Cl.: C07C 213/08, C07C 209/10

(54) **Verfahren zur Herstellung N-substituierter Nitro-p-phenylendiamine**
Process for the preparation of N-substituted nitro-p-phenylenediamines
Procédé pour la préparation de p-phénylènediamine N-substitués

(30) Priorität: 14.12.1991 DE 4141369
(43) Veröffentlichungstag der Anmeldung: 23.06.1993
(73) Patentinhaber: HOECHST AKTIENGESELLSCHAFT, D-65926 Frankfurt am Main (DE)
(72) Erfinder: Steckelberg, Willi, Dr., W-6238 Hofheim a. Ts. (DE); Koch, Peter, Dr., W-6053 Obertshausen (DE); Müller, Rolf, Dr., W-6367 Karben 4 (DE)

(56) Entgegenhaltungen:
- EP-A- 0 226 973
- GB-A- 1 206 491
- GB-A- 2 164 656

## Beschreibung

Die vorliegende Erfindung betrifft ein Verfahren zur Herstellung N-substituierter Nitro-p-phenylendiamine. N-substituierte Nitro-p-phenylendiamine sind bekannt und werden als Farbstoffe, insbesondere zum Färben menschlicher Haare, verwendet (siehe z.B. US 27 50 327, US 3,088,978, US 3,168,442, US 3,088,877, US 3,119,867, US 3,088,878, US 3,274,249, GB 1,206,491, EP 226 973 sowie DE 35 34 369).

In der Literatur sind bereits mehrere Verfahren zu ihrer Herstellung bekannt.

Beispielsweise können gemäß GB 955,743 und US 3,168,442 N¹-(Hydroxyalkyl)-2-nitro-1,4-phenylendiamine durch partielle Reduktion von 2,4-Dinitro-1-hydroxyalkylaminobenzolen erhalten werden. Nachteilig an diesem Verfahren ist, daß Isomerengemische sowie komplett reduzierte Triaminobenzole als Nebenprodukte entstehen, so daß eine Auftrennung Schwierigkeiten bereitet.

Gemäß DE 39 31 836 wird 2-Nitro-1,4-phenylendiamin zunächst in 4-Stellung acetyliert, dann die 1-Position mit einem Chloralkylchlorformiat in das korrespondierende Carbamat überführt und dieses zur N¹-Hydroxyalkyl-Verbindung umgelagert und verseift. Verseifung der 4-Acetamid-Funktion führt schließlich zur Zielverbindung. Nachteilig an diesem Verfahren ist, daß drei Reaktionsstufen in Gemischen aus organischem Lösungsmittel und Wasser ausgeführt werden. Dies bedeutet, daß die Lösungsmittel aufwendig regeneriert werden müssen. Darüberhinaus verlaufen die einzelnen Schritte mit unbefriedigender Selektivität, so daß nur ungenügende Produktreinheiten erzielt werden.

Es ist auch bereits bekannt, N-substituierte Nitro-p-phenylendiamine durch Umsetzung von 4-Fluor-3-nitroanilinen mit Aminen herzustellen (beispielsweise US 3,632,582, GB 1 206 491 und DE 17 68 999). Um eine möglichst vollständige Umsetzung zu erreichen, arbeiten diese Verfahren mit einem großen Aminüberschuß. Dies bringt erhebliche ökologische Belastungen mit sich, zumal eine Regenerierung der überschüssigen Amine aus ökonomischer Sicht nicht lohnend ist. Zudem liefern auch diese Verfahren Produkte ungenügender Reinheit und zusätzlich vergleichsweise geringe Ausbeuten.

Aufgabe vorliegender Erfindung ist es, ein sowohl aus ökologischer als auch aus ökonomischer Sicht akzeptables Verfahren zur Herstellung N-substituierter Nitro-p-phenylendiamine bereitzustellen.

Diese Aufgabe wird überraschenderweise gelöst durch ein Verfahren zur Herstellung von Verbindungen der allgemeinen Formel I 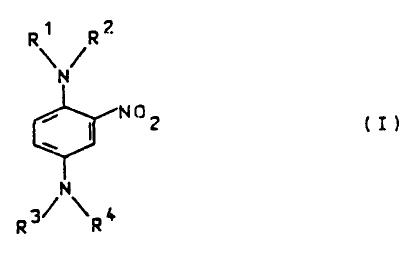
worin
R¹ und R² unabhängig voneinander Wasserstoff, (C₁-C₄)-Alkyl, (C₁-C₄)-Hydroxyalkyl oder (C₅-C₆)-Cycloalkyl bedeuten oder gemeinsam mit dem sie tragenden Stickstoffatom (C₄-C₆)-Heterocyclyl bilden und
R³ und R⁴ unabhängig voneinander Wasserstoff, (C₁-C₄)-Alkyl, (C₁-C₄)-Hydroxyalkyl, (C₁-C₂)-Alkylcarbonyl oder Tosyl
bedeuten, durch Umsetzung einer Verbindung der allgemeinen Formel II 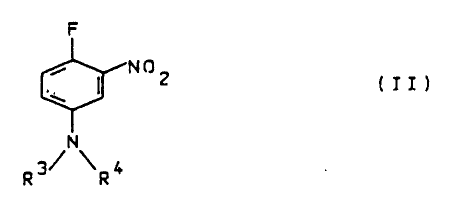
mit einem Amin der allgemeinen Formel III 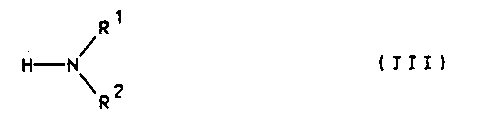
wobei R¹, R², R³ und R⁴ wie oben angegeben definiert sind, dadurch gekennzeichnet, daß in Gegenwart eines Alkalihydroxids gearbeitet und das Amin der allgemeinen Formel III in Mengen von 1,4 bis 1,8 Mol pro Mol Verbindung der allgemeinen Formel II eingesetzt wird.

Die genannten (C₁-C₄)-Alkylgruppen können geradkettig oder verzweigt sein und bedeuten Methyl, Ethyl, i-Propyl, n-Propyl, n-Butyl, i-Butyl, sek-Butyl und tert-Butyl. Analoges gilt für die genannten (C₁-C₄)-Hydroxyalkylgruppen, wobei die Hydroxyethyl-, 3-Hydroxypropyl- und 2-Hydroxypropylgruppen bevorzugt sind (C₅-C₆)-Cycloalkyl ist bevorzugt Cyclohexyl. (C₄-C₆)-Heterocyclyl ist bevorzugt Pyrrolidinyl, Piperidinyl oder Morpholinyl. Für (C₁-C₂)-Alkylcarbonyl steht bevorzugt die Acetylgruppe.

Bevorzugte Verbindungen der allgemeinen Formel II sind beispielsweise 4-Fluor-3-nitroanilin, N,N-Bis-(2-hydroxyethyl)-4-fluor-3-nitroanilin, N-Acetyl-4-fluor-3-nitroanilin und N-Tosyl-4-fluor-3-nitroanilin. Besonders bevorzugt sind 4-Fluor-3-nitroanilin und N,N-Bis(2-hydroxyethyl)-4-fluor-3-nitroanilin.

Bevorzugte Amine der allgemeinen Formel III sind beispielsweise Ethanolamin, Diethanolamin, 3-Hydroxypropylamin, 2-Hydroxypropylamin, Methylamin, Dimethylamin, Ethylamin, i-Propylamin, t-Butylamin, Cyclohexylamin, Pyrrolidin, Piperidin und Morpholin. Besonders bevorzugt sind Ethanolamin, 2-Hydroxypropylamin und 3-Hydroxypropylamin.

Bevorzugte Verbindungen der allgemeinen Formel I sind N¹-(2-Hydroxyethyl)-2-nitro-1,4-phenylendiamin und N¹,N⁴,N⁴-Tris-(2-hydroxyethyl)-2-nitro-1,4-phenylendiamin.

Alkalihydroxide sind bevorzugt Kaliumhydroxid und Natriumhydroxid, die zweckmäßigerweise in Form ihrer wäßrigen Lösungen, also z.B. als 50 %ige Natron- bzw. Kalilauge, eingesetzt werden.

Die Alkalihydroxide werden bevorzugt in Mengen von 0,6 bis 1,2, besonders bevorzugt von 0,7 bis 1,1 Mol pro Mol Verbindung der allgemeinen Formel II eingesetzt. Dabei können sie kontinuierlich über einen bestimmten Zeitraum oder pH-gesteuert zudosiert werden. Der pH-Wert wird dabei bevorzugt zwischen 8,5 und 9,5 gehalten.

Das Amin der allgemeinen Formel III wird bevorzugt in Mengen von 1,5 bis 1,7 Mol pro Mol Verbindung der allgemeinen Formel II eingesetzt.

Als Reaktionsmedium wird bevorzugt Wasser eingesetzt. Es ist dabei besonders bevorzugt als Reaktionsmedium das Waschwasser einer vorhergehenden erfindungsgemäßen Umsetzung einzusetzen. Dies kann ohne zusätzliche Reinigung mindestens für fünf weitere Reaktionsansätze verwendet werden.

Bevorzugt wird die erfindungsgemäße Reaktion bei Temperaturen zwischen Raumtemperatur und 120°C, besonders bevorzugt bei 80 - 100°C ausgeführt.

Die Ausgangsverbindungen der allgemeinen Formel II sowie die Amine der allgemeinen Formel III sind bekannt. Sie können im Handel erworben oder nach bekannten Verfahren hergestellt werden.

Das erfindungsgemäße Verfahren liefert die Verbindungen der allgemeinen Formel I in höheren Ausbeuten und in höherer Reinheit als die Verfahren des Standes der Technik. Dies ist umso überraschender, als der Fachmann aufgrund der Verwendung von Alkalihydroxid eine Substitution des Fluors durch Hydroxy, d.h. das Entstehen von Phenolen, erwartet hätte. In der Literatur (Synth. Commun. 13 (1983) 233) ist nämlich die Synthese von Nitrophenolen durch Umsetzung von Fluornitrobenzolen mit Natronlauge beschrieben. Phenole entstehen aber bei der erfindungsgemäßen Umsetzung nicht.

### Beispiel 1

156 g (1 Mol) 4-Fluor-3-nitroanilin und 97,6 g (1,6 Mol) Ethanolamin werden in 500 ml Wasser kauf 100°C erwärmt. Wenn die Farbe der Mischung von gelb nach rot umgeschlagen ist, werden während 5 h kontinuierlich 64 g (0,8 Mol) 50 %ige Natronlauge zudosiert, so daß der pH-Wert zwischen 8,5 und 9,5 gehalten wird. Es wird noch 1 h bei 100°C gerührt, auf Raumtemperatur abgekühlt und der entstandene Niederschlag abgesaugt und mit 500 ml Wasser in mehreren Portionen gewaschen.

| | |
|---|---|
| Ausbeute: | 90 % (177 g bronzefarbene Kristalle), |
| Fp.: | 121 - 123°C; |
| Reingehalt: | 95 - 96 % (spektralphotometrische Bestimmung) |
| Reaktionsvolumen: | 0,7 l/Mol |
| Abwassermenge: | 0,7 l/Mol |
| CSB: | 42 g/l |

Zum Vergleich wurde Beispiel 3 der US 3,632,582 nachgearbeitet und dabei folgende Werte erhalten:

| | |
|---|---|
| Ausbeute: | 73 % |
| Fp.: | 118°C |
| Reingehalt: | 82 %(spektralphotometrische Bestimmung) |
| Reaktionsvolumen: | 1,3 l/Mol |
| Abwassermenge: | 1,3 l/Mol |
| CSB: | 104 g/l |

### Beispiel 2

122 g (0,5 Mol) N,N-Bis(2-hydroxyethyl)-4-fluor-3-nitroanilin und 48,8 g (0,8 Mol) Ethanolamin werden in 250 ml Wasser auf 100°C erwärmt. Wenn die Farbe der Mischung von gelb nach violett umgeschlagen ist, werden in 5 h kontinuierlich 32 g (0,4 Mol) 50 %ige Natronlauge so zudosiert, daß der pH-Wert zwischen 8,5 und 9,5 gehalten wird. Es wird noch 1 h bei 100°C gerührt, auf 10°C abgekühlt, das Produkt abgesaugt und mit 250 ml Wasser in mehreren Portionen gewaschen.

| | |
|---|---|
| Ausbeute: | 85 % (121 g), |
| Fp.: | 103 - 104°C; |
| Reingehalt: | 96 %(spektralphotometrische Bestimmung) |
| Reaktionsvolumen: | 0,7 l/Mol |

Zum Vergleich wurde Beispiel 14 der US 3,632,582 nachgearbeitet und dabei folgende Werte erhalten:

| | |
|---|---|
| Ausbeute: | 79 % |
| Fp.: | 100 - 101°C |
| Reingehalt: | 94 %(spektralphotometrische Bestimmung) |
| Reaktionsvolumen: | 1,3 l/Mol |

## Patentansprüche

1. Verfahren zur Herstellung von Verbindungen der allgemeinen Formel I 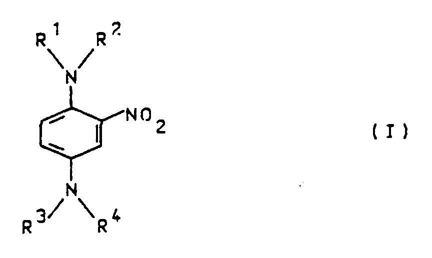 worin
R¹ und R² unabhängig voneinander Wasserstoff, (C₁-C₄)-Alkyl, (C₁-C₄)-Hydroxyalkyl oder (C₅-C₆)-Cycloalkyl bedeuten oder gemeinsam mit dem sie tragenden Stickstoffatom (C₄-C₆)-Heterocyclyl bilden und
R³ und R⁴ unabhängig voneinander Wasserstoff, (C₁-C₄)-Alkyl, (C₁-C₄)-Hydroxyalkyl, (C₁-C₂)-Alkylcarbonyl oder Tosyl
bedeuten, durch Umsetzung einer Verbindung der allgemeinen Formel II 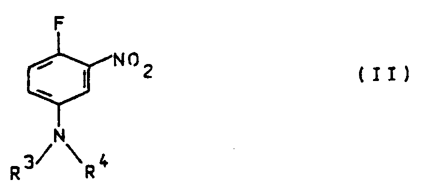 mit einem Amin der allgemeinen Formel III 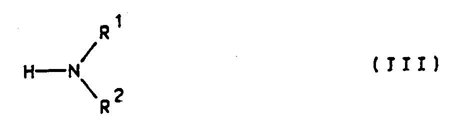 wobei R¹, R², R³ und R⁴ wie oben angegeben definiert sind, dadurch gekennzeichnet, daß in Gegenwart eines Alkalihydroxids gearbeitet und das Amin der allgemeinen Formel III in Mengen von 1,4 bis 1,8 Mol pro Mol Verbindung der allgemeinen Formel II eingesetzt wird.

2. Verfahren gemäß Anspruch 1, dadurch gekennzeichnet, daß N¹-(2-Hydroxyethyl)-2-nitro-1,4-phenylendiamin hergestellt wird.

3. Verfahren gemäß Anspruch 1, dadurch gekennzeichnet, daß N¹,N⁴,N⁴-Tris-(2-hydroxyethyl)-2-nitro-1,4-phenylendiamin hergestellt wird.

4. Verfahren gemäß einem oder mehreren der Ansprüche 1 bis 3, dadurch gekennzeichnet, daß als Alkalihydroxid Kaliumhydroxid oder Natriumhydroxid in Form ihrer wäßrigen Lösungen eingesetzt wird.

5. Verfahren gemäß einem oder mehreren der Ansprüche 1 bis 4, dadurch gekennzeichnet, daß als Reaktionsmedium Wasser eingesetzt wird.

6. Verfahren gemäß Anspruch 5, dadurch gekennzeichnet, daß als Reaktionsmedium das Waschwasser einer vorhergehenden Umsetzung gemäß Anspruch 1 eingesetzt wird.

## Claims

1. Process for the preparation of compounds of the general formula I 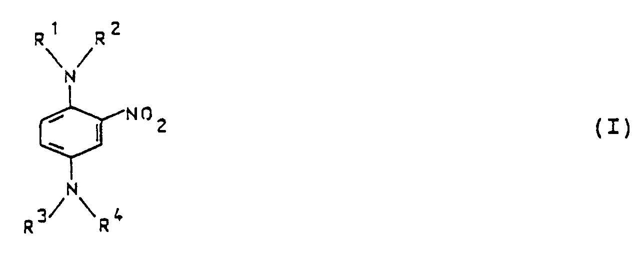 wherein
R¹ and R² independently of one another denote hydrogen, (C₁-C₄)-alkyl, (C₁-C₄)-hydroxyalkyl or (C₅-C₆)-cycloalkyl, or, together with the nitrogen atom carrying them, form (C₄-C₆)-heterocyclyl and
R³ and R⁴ independently of one another denote hydrogen, (C₁-C₄)-alkyl, (C₁-C₄)-hydroxyalkyl, (C₁-C₂)-alkylcarbonyl or tosyl, by reaction of a compound of the general formula II 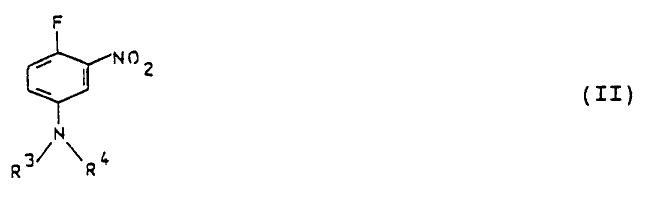 with an amine of the general formula III 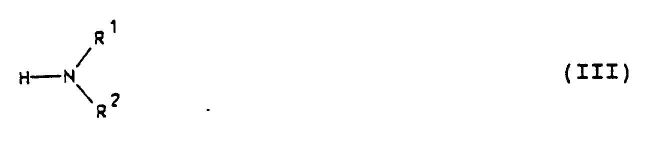 wherein R¹, R², R³ and R⁴ are defined as stated above, characterised in that the reaction is carried out in the presence of an alkali metal hydroxide, and the amine of the general formula III is employed in amounts of 1.4 to 1.8 mol per mol of compound of the general formula II.

2. Process according to Claim 1, characterised in that N¹-(2-hydroxyethyl)-2-nitro-1,4-phenylenediamine is prepared.

3. Process according to Claim 1, characterised in that N¹,N⁴,N⁴-tris-(2-hydroxyethyl)-2-nitro-1,4-phenylenediamine is prepared.

4. Process according to one or more of Claims 1 to 3, characterised in that potassium hydroxide or sodium hydroxide, in the form of their aqueous solutions, is employed as the alkali metal hydroxide.

5. Process according to one or more of Claims 1 to 4, characterised in that water is employed as the reaction medium.

6. Process according to Claim 5, characterised in that the washwater of a previous reaction according to Claim 1 is employed as the reaction medium.

## Revendications

1. Procédé de préparation de composés de formule générale I 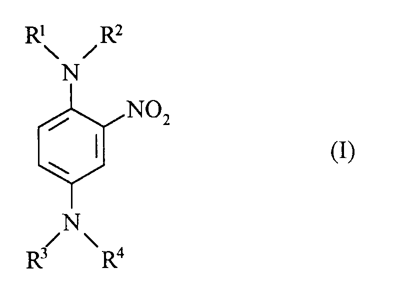 dans laquelle
R¹ et R² représentent indépendamment l'un de l'autre un hydrogène ou un groupe alkyle en C₁-C₄, hydroxyalkyle en C₁-C₄ ou cycloalkyle en C₅-C₆, ou forment ensemble avec l'atome d'azote auquel ils sont liés un groupe hétérocyclique en C₄-C₆, et
R³ et R⁴ représentent indépendamment l'un de l'autre un hydrogène ou un groupe alkyle en C₁-C₄, hydroxyalkyle en C₁-C₄, (alkyl en C₁-C₂)carbonyle ou tosyle,
par réaction d'un composé de formule générale II 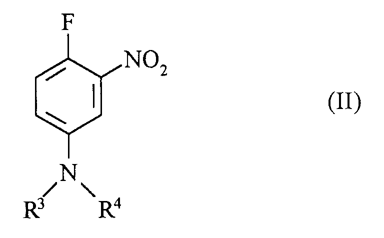 avec une amine de formule générale III 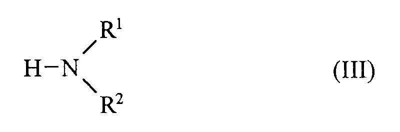 R¹, R², R³ et R⁴ ayant la définition donnée ci-dessus, caractérisé en ce que l'on travaille en présence d'un hydroxyde alcalin et en ce que l'on utilise l'amine de formule générale III en des quantités de 1,4 à 1,8 mole par mole de composé de formule générale II.

2. Procédé selon la revendication 1, caractérisé en ce que l'on prépare la N¹-(2-hydroxyéthyl)-2-nitro 1,4-phénylènediamine.

3. Procédé selon la revendication 1, caractérisé en ce que l'on prépare la N¹,N⁴,N⁴-tris(2-hydroxyéthyl)-2-nitro-1,4-phénylènediamme.

4. Procédé selon l'une ou plusieurs des revendications 1 à 3, caractérisé en ce que l'on utilise comme hydroxyde alcalin l'hydroxyde de potassium ou l'hydroxyde de sodium sous forme de leurs solutions aqueuses.

5. Procédé selon l'une ou plusieurs des revendications 1 à 4, caractérisé en ce que l'on utilise de l'eau comme milieu réactionnel.

6. Procédé selon la revendication 5, caractérisé en ce que l'on utilise comme milieu réactionnel l'eau de lavage d'une réaction précédente selon la revendication 1.
